# EUROPEAN PATENT SPECIFICATION

(11) **EP 1 801 238 B1**
(45) Date of publication and mention of the grant of the patent: **07.07.2010**
(21) Application number: 06120835.1
(22) Date of filing: 18.09.2006
(51) Int. Cl.: C12Q 1/68, C12M 1/34, G01N 33/543, B01J 19/00, B05D 1/00

(54) **Microarray substrate, method of manufacturing the same, microarray using the microarray substrate, and method of analyzing target biomolecule using the microarray**
Mikroarray-Substrat, Verfahren zu dessen Herstellung, Mikroarray mit dem Mikroarray-Substrat und Verfahren zur Analyse von Zielmolekülen unter Verwendung des Mikroarrays
Substrat à microréseau, procédé de fabrication de celui-ci, microréseau utilisant le substrat à microréseau et procédé d'analyse d'une biomolécule cible à l'aide du microréseau

(30) Priority: 20.12.2005 KR 20050126263
(43) Date of publication of application: 27.06.2007
(73) Proprietor: Samsung Electronics Co., Ltd., Suwon-si, Gyeonggi-Do (KR)
(72) Inventor: Park, Jong-myeon, Songpa-gu, Seoul (KR); Peak, Sang-hyun, Yangcheon-gu, Seoul (KR)
(74) Representative: Grünecker, Kinkeldey, Stockmair & Schwanhäusser Anwaltssozietät

(56) References cited:
- EP-A1- 1 489 415
- WO-A1-03/020978
- WO-A2-2006/058237
- US-A- 5 624 711
- US-A1- 2002 128 234
- SPERLING C ET AL: "Covalently immobilized thrombomodulin inhibits coagulation and complement activation of artificial surfaces in vitro" BIOMATERIALS, ELSEVIER SCIENCE PUBLISHERS BV., BARKING, GB, vol. 25, no. 21, September 2004 (2004-09), pages 5101-5113, XP004504200 ISSN: 0142-9612

<110> Samsung Electronics Co., Ltd.
<120> A substrate used in a macroarray and a method for preparing the same
<130> PN065234
<160> 9
<170> KopatentIn 1.71
<210> 1
   <211> 15
   <212> DNA
   <213> Artificial Sequence
<220>
   <223> probe
<400>
   tgttctcttg tcttg 15
<210> 2
   <211> 15
   <212> DNA
   <213> Artificial Sequence
<220>
   <223> target DNA
<400> 2
   caagacaaga gaaca 15
<210> 3
   <211> 22
   <212> DNA .
   <213> Artificial Sequence
<220>
   <223> probe
<400> 3
   acggagttac aaaggacgac at 22
<210> 4
   <211> 22
   <212> DNA
   <213> Artificial Sequence
<220>
   <223> probe
<400> 4
   aaaggacgac attagacgaa tc 22
<210> 5
   <211> 25
   <212> DNA
   <213> Artificial Sequence
<220>
   <223> probe
<400> 5
   aacatatgtg taagtaactg tgcac 25
<210> 6
   <211> 20
   <212> DNA
   <213> Artificial Sequence
<220>
   <223> primer
<400> 6
   yccakactcc tacgggaggc 20
<210> 7
   <211> 22
   <212> DNA
   <213> Artificial Sequence
<220>
   <223> primer
<400> 7
   gtgccagcag yygcggtaat ac 22
<210> 8
   <211> 22
   <212> DNA
   <213> Artificial Sequence
<220>
   <223> primer
<400> 8
   tagcatatca gaaggcacac cc 22
<210> 9
   <211> 23
   <212> DNA
   <213> Artificial Sequence
<220>
   <223> primer
<400> 9
   aatgttgtct ctcttgagtg gat 23

## Description

### 1. Field of the Invention

The present invention relates to a microarray substrate and a method of manufacturing the same. More particularly, the present invention relates to a microarray substrate in which a functionalized (poly)ethyleneglycol compound is coated on a solid support having a polyanhydride surface, and a method of manufacturing the same.

### 2. Description of the Related Art

A microarray refers to a high-density array of predetermined molecules immobilized in predetermined regions of a substrate. The microarray can be classified into polynucleotide microarray, protein microarray, etc. The term "polynucleotide microarray" refers to a high-density array of two or more groups of polynucleotides immobilized on a substrate. Here, each of the two or more groups of the polynucleotides is a microarray immobilized on predetermined regions of the substrate. The microarray is well known in the art. Examples of such microarrays are disclosed in U.S. Pat. No. 5,445,934 and No. 5,744,305.

The immobilization of polynucleotides on a solid substrate can be achieved by direct synthesis of polynucleotides on a solid substrate or by immobilization (spotting) of previously synthesized polynucleotides on predetermined regions of a solid substrate. Illustrative methods for manufacturing such polynucleotide microarrays are disclosed in U.S. Pat. No. 5,744,305, No: 5,143,854, and No. 5,424,186. A spotting method has been widely used for the immobilization of biomolecules on a solid substrate. However, the spotting method has been used for covalent attachment of biomolecules on a solid substrate. For example, there has been widely used a method of immobilizing biomolecules on a solid substrate which includes: activating a surface of a solid substrate with a nucleophilic functional group (e.g., an amino group), coupling biomolecules (e.g., polynucleotides) activated with a good leaving group to the surface-activated solid substrate, and removing unreacted reactants.

In addition, a microarray using hydrophilic polyethyleneglycol has been reported. For example, U.S. Pat. Laid-Open Publication NO. 2003-0108917 A1 discloses a method of manufacturing a microarray wherein probe polynucleotides are immobilized on a hydrogel composed of a star-like polyethyleneglycol derivative having an epoxy end-functional group.

US-A-5,624,711 discloses methods and derivatized supports which are useful in solid-phase synthesis of peptides oligonucleotides as well as arrays of ligands. A polymer, for example, poly(ethylene/maleic anhydride) can be linked to a solid substrate whose surface is derivatized with, e.g. aminoalkyltrialkoxysilanes.

WO 2006/058237 A2 discloses a polymer-coated substrate for binding biomolecules, wherein the polymer may be poly(ethylene-alt-maleic anhydride) or poly(triethyleneglycol methylvinyl ether-co-maleic anhydride). This polymer is attached to the substrate via a tie layer, for example, via 3-aminopropyltrimethoxysilane.

EP-A1-1 489 415 refers to a substrate having a brush-like structured surface of poly(ethylene oxide) having elevated density. A functionalized poly(ethylene glycol) (PEG) is linked to the solid support by a trialkoxysilyl group, wherein the functional group of the PEG may be an aldehyde, amino or carboxyl group.

US 2002/128234 A1 discloses a substrate coated with a polymer having a polyionic backbone, such as poly(amino acid), with grafted PEG-side chains and analyte-specific side chains having functional groups, for example, a substrate is coated with a co-polymer of poly-L-lysine-PEG or poly-A-glutamic acid-PEG.

WO 03/020978 A1 discloses star-like PEG-derivatives and their use in the manufacture of hydrogel biochips. Sperling, C. et al.: "Covalently immobilized thrombomodulin inhibits coagulation and complement activation of artificial surfaces in vitro" BIOMATERIALS, ELSEVIER SCIENCE PUBLISHERS BV., BARKING, GB, vol. 25, no. 21, September 2004 (2004-09), pages 5101-5113 refers to a hemocompatible surface inhibiting coagulation and complement activation and discloses a glass surface treated with 3-aminopropyl-dimethylethoxysilane and coated with poly(octadecene-alt-maleic anhydride). The anhydride group is then converted with diaminobutane to generate many amino groups. PEG-diacid is bound to the amino group and finally thrombomodulin is immobilized to the free carboxyl group of the modified surface.

### SUMMARY OF THE INVENTION

The present invention provides a microarray substrate that enhances the immobilization efficiency of a probe biomolecule and reduces non-specific binding of a target biomolecule and a protein on a substrate.

The present invention also provides a method of manufacturing the microarray substrate.

The present invention also provides a microarray wherein a probe biomolecule is immobilized on the microarray substrate and a method of analyzing a target biomolecule using the microarray.

According to an aspect of the present invention, there is provided a microarray substrate according to claim 1.

According to another aspect of the present invention, there is provided a method of manufacturing a microarray substrate, according to claim 10.

According to a microarray substrate of the present invention, the immobilization efficiency of a probe biomolecule is enhanced and non-specific binding of a target biomolecule and a protein on the microarray substrate-is blocked. Thus, after a probe biomolecule is immobilized on the microarray substrate, no further post-treatment on the microarray substrate is required, and a crude PCR sample can be directly applied to the microarray substrate, thereby ensuring process simplicity and cost-effectiveness in chip production and biomolecule assay.

### BRIEF DESCRIPTION OF THE DRAWINGS

The above and other features and advantages of the present invention will become more apparent by describing in detail exemplary embodiments thereof with reference to the attached drawings in which:
FIG. 1 illustrates immobilization of poly(ethylene-alt-maleic anhydride) on a solid support coated with gamma-aminopropyltriethoxysilane (GAPS);
FIG. 2 illustrates incorporation of an amino group into polyethyleneglycol (PEG);
FIGS. 3A through 3D are ¹H NMR spectra of a compound of Formula 1 which is a starting compound for incorporation of an amino group onto PEG (FIG. 3A), compounds of Formulae 2 and 3 which are intermediates for the incorporation of the amino group onto the PEG (FIGS. 3B and 3C), and a compound of Formula 4 which is a product compound of the incorporation of the amino group onto the PEG (FIG. 3D), respectively;
FIG. 4 illustrates coating of an amino-functionalized PEG on a solid support having a poly(ethylene-alt-maleic anhydride) surface;
FIG. 5A shows fluorescence image data obtained after a control microarray 1 and a test microarray 1 according to the present invention are hybridized with target DNAs and FIG. 5B illustrates fluorescence intensity data obtained after the hybridization of FIG. 5A (fluorescence intensity is expressed as RFU);
FIG. 6 illustrates fluorescence intensity data obtained after FITC-labeled BSA is applied to a test substrate according to the present invention and a control substrate (fluorescence intensity is expressed as RFU); and
FIG. 7 shows fluorescence image data obtained after a control microarray 2 and a test microarray 2 according to the present invention, which are manufactured without performing a process for protecting an unreacted amino group on a substrate after probe immobilization, are hybridized with a target DNA sample which is not purified after amplification.

### DETAILED DESCRIPTION OF THE INVENTION

The present invention will now be described more fully with reference to the accompanying drawings, in which exemplary embodiments of the invention are shown.

The present invention provides a microarray substrate wherein a functionalized (poly)ethyleneglycol compound is coated on a solid support surface-modified with polyanhydride.

In the present invention, a material of the solid support is not particularly limited provided that it is a solid phase material capable of providing a surface. For example, the solid support may be a plastic substrate made of polyethylene, polypropylene, polystyrene, polyurethane, or polyolefin, a glass substrate, a silicone wafer, or a modified substrate thereof. In the present invention, the solid support may have a functional group capable of acting as a hydrogen bond donor, such as an amino group, a thiol group, or a hydroxy group. Thus, such a functional group may be already present on the solid support itself due to intrinsic characteristics of a material of the solid support or may be incorporated onto the solid support using a chemical or physical treatment (e.g., coating).

In the present invention, the solid support may be coated with an amino group-containing compound. The amino group-containing compound may be gamma-aminopropyltriethoxysilane (GAPS), gamma-aminopropyldiethoxysilane (GAPDES), or an aminohexyl group, but the present invention is not limited to the illustrated examples. Preferably, the solid support may be a solid support coated with GAPS.

The polyanhydride used herein may be poly(ethylene-graft-maleic anhydride), poly(isobutylene-alt-maleic anhydride), poly[(isobutylene-alt-malemide)-co-isobutylene-alt-maleic anhydride], etc. Poly(ethylene-*alt*-maleic anhydride) is preferred. The poly(ethylene-*alt*-maleic anhydride) may have a molecular weight from 128 to 10,000,000, and most preferably from 100,000 to 500,000.

In the present invention, the solid support surface-modified with the polyanhydride is coated with the functionalized (poly)ethyleneglycol compound. Here, the (poly)ethyleneglycol compound is functionalized with an amino group. The (poly)ethyleneglycol compound may have a molecular weight from 60 to 10,000,000, preferably from 200 to 1,000,000, and more preferably from 200 to 10,000.

The (poly)ethyleneglycol compound may be a star-like or linear molecule.

The present invention also provides a method of manufacturing a microarray substrate, according to claim 10.

In the method of manufacturing the microarray substrate according to the present invention, first, the solid support coated with the amino group-containing compound is prepared. The amino group-containing compound may be GAPS, GAPDES, or an aminohexyl group. GAPS is most preferred. In this operation, the solid support may be a commercially available amino group-containing solid support or a solid support obtained by incorporating an amino group onto an unmodified solid support using a chemical or physical treatment (e.g., coating).

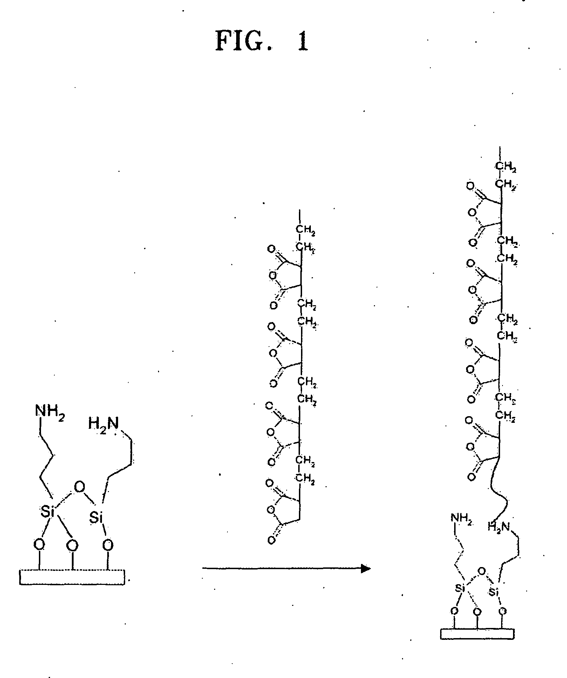

After preparing the solid support coated with the amino group-containing compound, the polyanhydride is immobilized on the surface of the solid support (see FIG. 1). The polyanhydride may be poly(ethylene-alt-maleic anhydride). Generally, the immobilization of the polyanhydride on the surface of the solid support is achieved by attaching the polyanhydride to the amino group present on the surface of the solid support.

After immobilizing the polyanhydride on the surface of the solid support, the solid support is surface-coated with the functionalized (poly)ethyleneglycol. The functionalized (poly)ethyleneglycol is amino-functionalized (poly)ethyleneglycol. For example, the amino-functionalized (poly)ethyleneglycol reacts with the polyanhydride surface of the polyanhydride-immobilized solid support. In detail, an anhydride group of the polyanhydride reacts with the amino group of the (poly)ethyleneglycol. As a result, many (poly)ethyleneglycols, amino groups, and- carboxyl groups are exposed on the surface of the microarray substrate of the present invention. The amino-functionalized (poly)ethyleneglycol can be synthesized using a method commonly known in the art. (Poly)ethyleneglycol having as many amino groups as possible is preferred.

The microarray substrate manufactured by the above-illustrated method has many amino groups on its surface, and thus, enhances the immobilization efficiency of probe biomolecules. Furthermore, the (poly)ethyleneglycols and carboxyl groups on the surface of the microarray substrate prevent non-specific adsorption of target biomolecules on other substrate regions except substrate regions immobilized with probe biomolecules under the pH condition for probe-target hybridization and non-specific adsorption of proteins on the microarray substrate. Thus, a hybridization process and a hybridization assay can be directly performed without performing a further post-treatment (e.g., background capping) after probe immobilization or a further purification after cell lysis and PCR, thereby ensuring process simplicity and cost-effectiveness in chip production and biomolecule assay.

The present invention also provides a microarray in which the microarray substrate is immobilized with a probe biomolecule and a method of analyzing a target biomolecule using the microarray.

In the present invention, a biomolecule is a compound derived from living organisms and may be selected from the group consisting of a nucleic acid, a protein, a polysaccharide, and a combination thereof. A nucleic acid is preferred. The nucleic acid may be DNA or RNA. In the present invention, the probe biomolecule immobilized on the microarray substrate is generally a biomolecule capable of specifically reacting with a target biomolecule. For example, when a nucleic acid is used as the probe biomolecule, the probe biomolecule can hybridize with a target nucleic acid having a nucleotide sequence complementary to the nucleotide sequence of the probe biomolecule. When a protein is used as the probe biomolecule, the probe biomolecule can specifically interact with a target protein through an antigen-antibody reaction, a ligand-receptor reaction, or an enzyme-substrate reaction. When a polysaccharide is used as the probe biomolecule, the probe biomolecule can specifically interact with a protein (e.g., lectin) or antibody recognizing a polysaccharide. The microarray according to the present invention can be used for various assays using a detection system capable of detecting a specific interaction between a probe biomolecule and a target molecule and an assay system capable of analyzing the detection result.

In the present invention, the concentration of the probe biomolecule may vary according to reaction conditions or the type of desired data. Thus, the concentration of the probe biomolecule is not particularly limited. In an embodiment of the present invention, when DNA is used as the probe biomolecule, the probe biomolecule may be used in the concentration of 20 to 100 µ M, but the present invention is not limited thereto.

In the present invention, the immobilization of the probe biomolecule on the microarray substrate can be performed by a conventional method used for manufacturing a DNA or protein microarray. Generally, a microarray can be manufactured using a photolithographic method. According to a photolithographic method, a polynucleotide microarray can be manufactured by repeatedly exposing a predetermined surface region of a substrate coated with a monomer protected by a removable protecting group to an energy source to remove the protecting group and coupling the deprotected monomer with another monomer protected by the removable protecting group. In this case, immobilization of a polynucleotide on the polynucleotide microarray can be achieved by synthesizing a polynucleotide by extending monomers of the polynucleotide one by one or by immobilizing a previously synthesized polynucleotide in a predetermined region (which is also called "spotting").

Hereinafter, the present invention will be described more specifically with reference to the following working examples.

### Example 1: Preparation of microarray substrate according to the present invention and control substrate

### (1) Manufacturing of microarray substrate according to the present invention

### (a) Immobilization of polyanhydride on surface of silicone wafer coated with GAPS

In order to manufacture a microarray substrate according to the present invention, first, a GAPS-coated silicone wafer (LG Siltron, Korea) was immersed in a solution of 200 mM (on a repeat unit basis) of poly(ethylene-alt-maleic anhydride) (molecular weight: 100,000 to 500,000) in N-methyl-2-pyrrolidone (NMP) at room temperature for one hour, washed with acetone and ethanol, and dried under vacuum (see FIG. 1).

### (b) Preparation of amino-functionalized polyethyleneglycol (PEG)

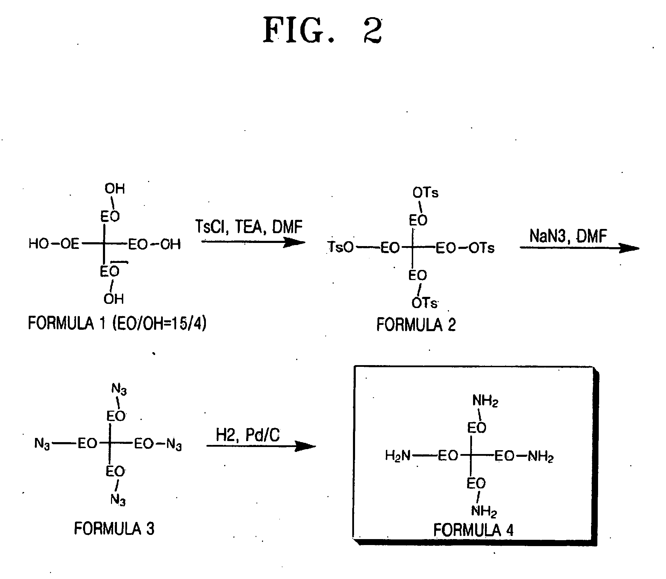

An amino-functionalized PEG compound wherein an amino group was incorporated into a PEG backbone was prepared as follows. A detailed preparation method thereof is illustrated in FIG. 2.

### (i) Preparation of compound of Formula 2

PEG (EO/OH=15/4) (1 eq.), which was a compound of Formula 1 used as a starting material, was incubated in the presence of triethylamine (TEA) (5g, 6 eq.), dimethylformamide (DMF) (20ml), and tosyl chloride (TsCl) (7.1g, 6 eq.) at 120°C for 3 hours. Removal of the starting material, PEG was identified by thin layer chromatography (TLC) (elution solvent: 10% methanol in CHCl₃).

The reaction solution was diluted with methylene chloride (10ml), transferred to a separatory funnel, and extracted with water to remove DMF and methylene chloride. Then, a solvent was removed using a rotary evaporator and the residue was purified by flash column chromatography. The flash column chromatography was performed by pressurizing a column packed with silica gel, loading the solvent-free residue on the silica gel, and pushing an elution solvent (10% methanol/90% chloroform) with a compressed air. As a result, a compound of Formula 2 was obtained.

### (ii) Preparation of compound of Formula 3

The compound of Formula 2 (1 eq.) and sodium azide (NaN₃) (10 eq.) were incubated in DMF in the presence of TEA (10 eq.) at 100°C overnight. Removal of the compound of Formula 2 was identified by TLC (elution solvent: 8% ethanol in CHCl₃).

The reaction solution was diluted with methylene chloride (10 ml), transferred to a separatory funnel, and extracted with water to remove DMF and methylene chloride. Then, a solvent was removed using a rotary evaporator and the residue was purified by flash column chromatography as described above to give a compound of Formula 3.

### (iii) Preparation of compound of Formula 4

The compound of Formula 3 (5 g) was incubated in a methanol solvent (20ml) in the presence of H₂ and a Pd/C catalyst (10 eq.). Removal of the compound of Formula 3 was identified by TLC (elution solvent: 8% methanol in CHCl₃) (Rf = 0).

The reaction solution was filtered through a Celite pad and a solvent was removed using a rotary evaporator to give a compound of Formula 4.

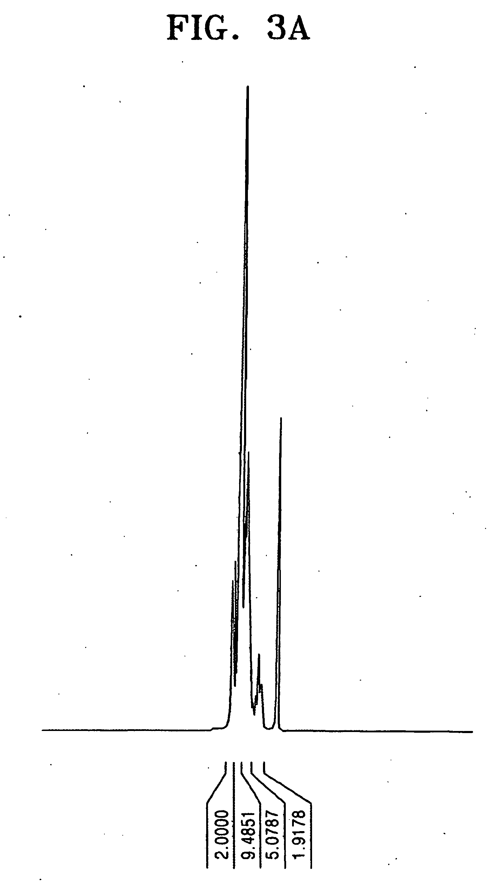

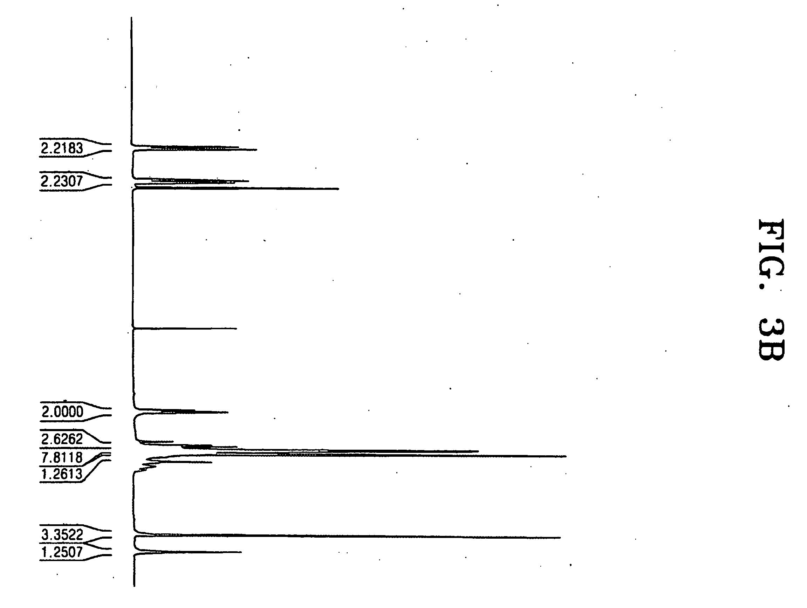

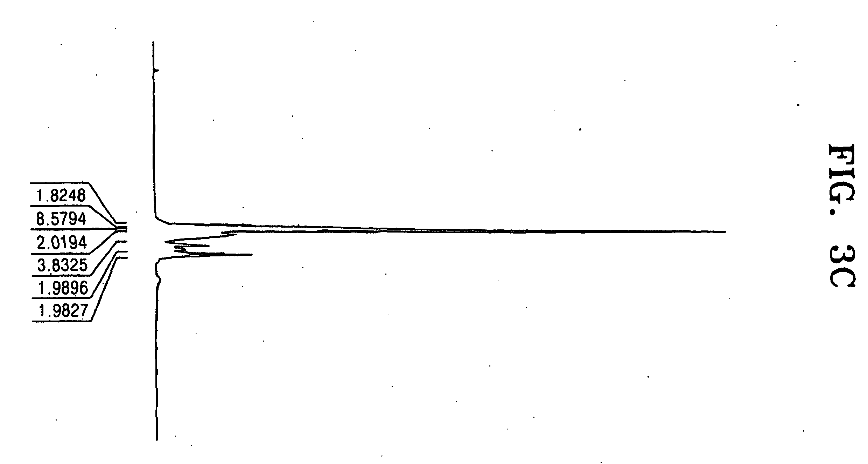

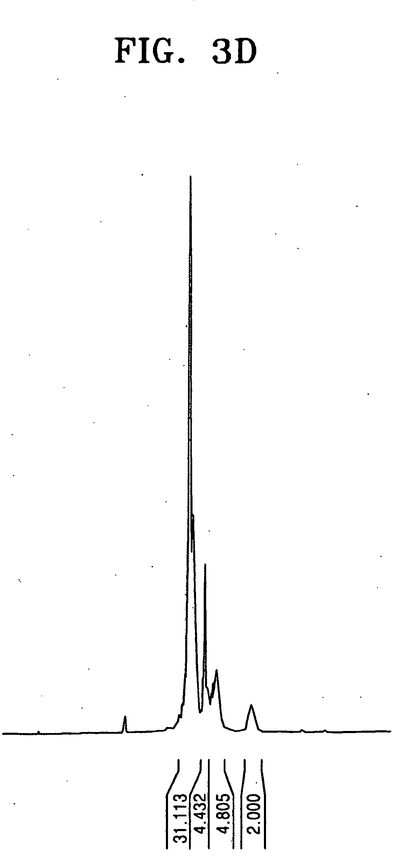

The compounds of Formulae 1 through 4 were identified by Bruker 500MHz ¹H NMR in a chloroform solvent, and the results are shown in FIGS. 3A through 3D, respectively.

### (c) Coating of polyanhydride-immobilized silicone wafer with amino-functionalized PEG

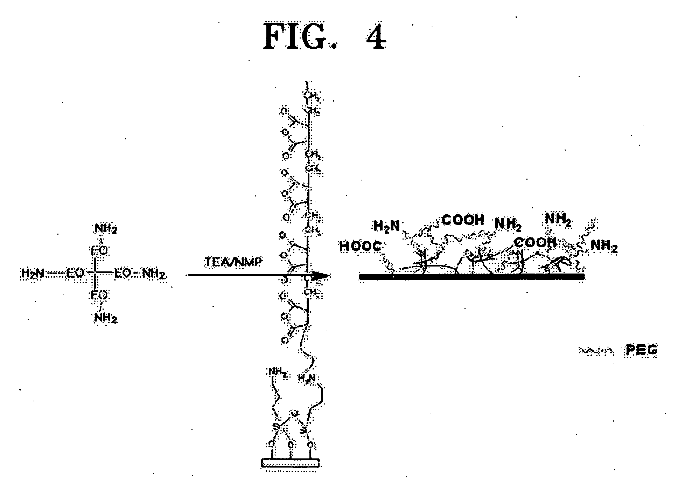

The polyanhydride-immobilized silicone substrate of (a) was immersed in a solution of the amino-functionalized PEG of (b) (200 mM) and water (300 mM) in NMP to induce hydrolysis, washed with ethanol and acetone, and dried, to give a microarray substrate according to the present invention (hereinafter, referred to as "test substrate") on which the amino group derived from the PEG and a carboxyl group were coated (see FIG. 4).

### (2) Preparation of control substrate

In the following working examples, a GAPS-coated silicone wafer was used as a control substrate.

### Example 2: Manufacturing of polynucleotide microarray wherein probe DNA is immobilized on substrate

A polynucleotide microarray wherein 5'-end functionalized DNA was arranged in two or more groups of spots of the test substrate prepared in (1) of Example 1 was manufactured as follows.

First, a spotting solution was prepared. The composition of the spotting solution was as follows: 50% formamide, 25% a solution of 9mM PEG (MW: 10,000) in NaHCO₃(0.1M, pH 10), and 25% a solution containing a probe DNA (SEQ ID NO: 1) having an amino (NH₂) group at the 5'-end. The final concentration of DNA was 20 µ M in distilled water.

The spotting solution thus prepared was spotted on the test substrate using a Pix5500 spotter (Cartesian), and incubated at 70°C, 40% humidity, for one hour, in a constant temperature and humidity chamber so that the probe DNA was immobilized on the test substrate. After the reaction terminated, the test substrate was washed with distilled water, incubated with anhydrous succinic acid (blocking agent) to protect an unreacted amino group, washed with ethanol, and spin-dried to obtain a polynucleotide microarray immobilized with the probe DNA, which was designated as "test microarray 1".

Meanwhile, a "control microarray 1" was manufactured in the same manner as above using the GAPS-coated silicone wafer prepared in (2) of Example 1.

### Example 3: Evaluation of immobilization efficiency of probe DNA

In Example 3, hybridization of the probe DNA immobilized on the test microarray 1 manufactured in Example 2 with a target DNA was performed and the hybridization results were detected to thereby determine the immobilization efficiency of the probe DNA on the test microarray 1.

For this, an oligonucleotide (SEQ ID NO: 2) having -NH₂-Cy3 at the 5'-end was used as a target DNA.

16µl of the target DNA (100 pM) was placed in a 1.5 ml tube, vortexed for 10 seconds, and centrifuged.

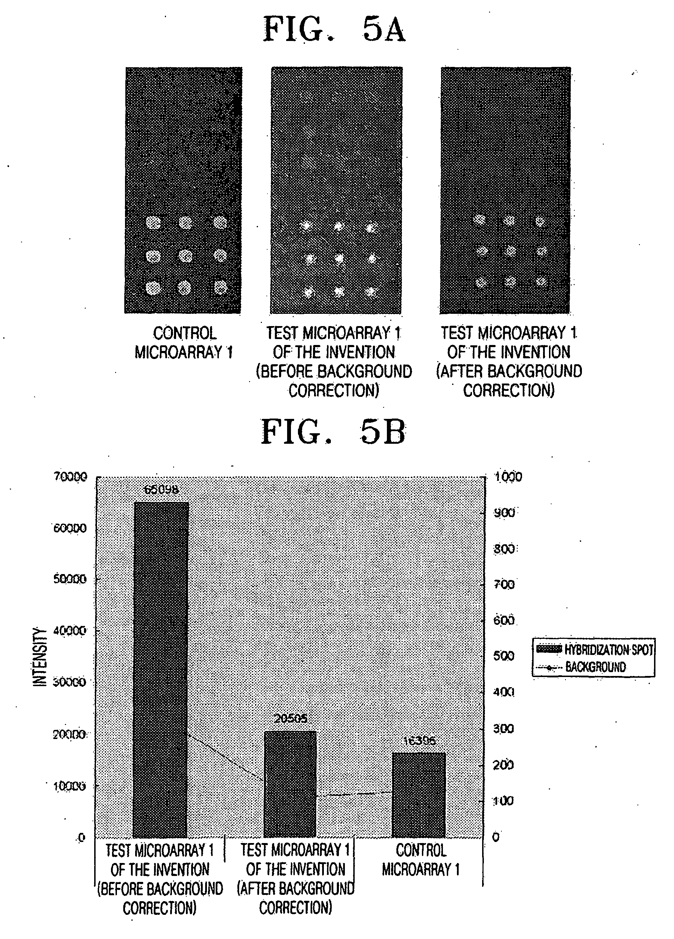

The target DNA was denatured in a 94°C heating block for 5 minutes and placed on ice. In this state, 16 µℓ of a hybridization buffer (a diluted solution of NaH₂PO₄H₂O 138g, NaCl 876g, 0.5M EDTA 200ml, and 10N NaOH 100ml) was added to the target DNA to reach a total volume of 32 µℓ. The reaction solution was vortexed for 10 seconds, centrifuged for 10 seconds, and added to the test microarray 1 and the control microarray 1. The test micoarray 1 and the control microarray 1 were incubated at 42°C for one hour, washed a washing solution I (1XSSPET) and a washing solution II (3XSSPET) for 5 minutes (for each), and dried. Fluorescence image data were acquired using a GenePix 4000B scanner (Axon Instruments) and analyzed using GenePix Pro software (Axon Instruments, Union City, CA). The fluorescence image data and the fluorescence intensity data are shown in FIGS. 5A and 5B, respectively. The fluorescence intensity was observed at 532 nm (PMT560).

Referring to FIGS. 5A and 5B, the immobilization efficiency of the probe DNA of the test microarray 1 before background correction was more than 300% higher than that of the control microarray 1. The immobilization efficiency of the probe DNA of the test microarray 1 after background correction was more than 30% higher than that of the control micoarray 1.

### Example 4: Non-specific binding test of protein on substrate according to the present invention

500 µℓ of a FITC-labeled bovine serum albumin (BSA) (standard protein) in aqueous solution (1 mg/ml) was added to the test substrate and the control substrate prepared in Example 1. The test substrate and the control substrate were incubated in a covered chamber at room temperature for 2 minutes and washed with distilled water. Fluorescence intensity from the test substrate and the control substrate was measured.

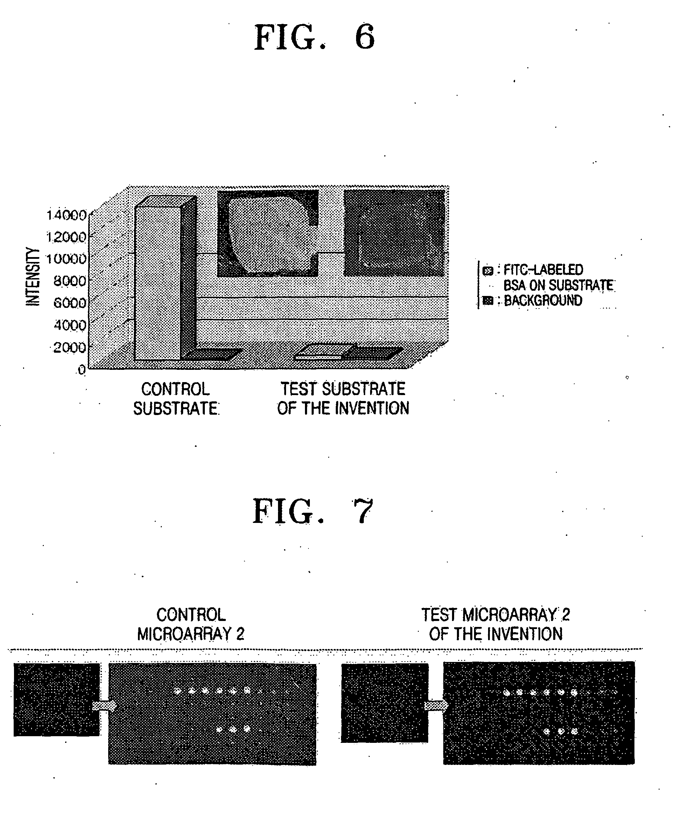

The fluorescence intensity was measured at 532 nm(PMT560) using a GenePix 4000B scanner (Axon Instruments) to acquire fluorescence images. The fluorescence images were analyzed using GenePix Pro software (Axon Instruments, Union City, CA). The results are presented in Table 1 below and FIG. 6.

**Table 1**

| | Control substrate | Test substrate |
|---|---|---|
| FITC-labeled BSA | 13924.93 | 465.37 |
| Background | 110 | 213 |

As shown in Table 1 and FIG. 6, non-specific binding of the protein on the test substrate of the invention was significantly reduced (at least 96% reduction) as compared to the control substrate.

### Example 5: Non-specific binding test of target DNA under the conditions of no purification after PCR and no post-treatment after probe immobilization

A test microarray 2 of the invention and a control microarray 2 were manufactured in the same manner as in Example 2 except that three types of oligonucleotides (SEQ ID NOS: 3 through 5) were used as probe DNAs and no process for protecting an unreacted amino group (protection of the amino group with anhydrous succinic acid (blocking agent)) after probe immobilization was performed.

Target DNAs were designed to have DNA sequences corresponding to 16S rRNA and 23S rRNA of *Staphylococcus aureus* which was a type of respiratory bacteria.

The target DNA corresponding to 16S rRNA was amplified by PCR using oligonucleotides having SEQ ID NOS: 6 and 7 as forward and reverse primers, and the target DNA corresponding to 23S rRNA was amplified by PCR using oligonucleotides having SEQ ID NOS: 8 and 9 as forward and reverse primers.

5'-ends of the amplified target DNAs were labeled with Cy5. Then, the Cy5-labeled target DNAs were hybridized on the test microarray 2 and the control microarray 2 without further purification. The hybridization was performed in the same manner as in Example 3.

Fluorescence images from the test microarray 2 before and after the hybridization were acquired at 532 nm using a GenePix 4000B scanner (Axon Instruments). The fluorescence images were analyzed using GenePix Pro software (Axon Instruments, Union City, CA). The results are presented in Table 2 below and FIG. 7.

**Table 2**

| | Control microarray 2 | | Test microarray 2 | |
|---|---|---|---|---|
| | Before hybridization | After hybridization | Before hybridization | After hybridization |
| Background | 61.11 | 154.85 | 63.31 | 82.56 |
| Increase (%) | | 154 | | 30 |

As shown in Table 2 and FIG. 7, when performing the hybridization with no protection of the unreacted amino group after probe immobilization, background emission from the control microarray 2 was increased by more than 150% as compared to that before the hybridization, whereas self-emission from the test microarray 2 was increased by only 30% as compared to that before the hybridization.

These results show that with respect to a microarray of the present invention, even when no process for protecting an unreacted amino group exposed on a surface of a microarray substrate after probe immobilization is performed, non-specific binding of a target DNA on the microarray substrate can be significantly reduced.

As described above, according to a microarray substrate of the present invention, the immobilization efficiency of a probe biomolecule is enhanced and non-specific binding of a target biomolecule and a protein on the microarray substrate is blocked. Thus, after a probe biomolecule is immobilized on the microarray substrate, no further post-treatment on the microarray substrate is required, and a crude PCR sample can be directly applied to the microarray substrate, thereby ensuring process simplicity and cost-effectiveness in chip production and biomolecule assay.

While the present invention has been particularly shown and described with reference to exemplary embodiments thereof, it will be understood by those of ordinary skill in the art that various changes in form and details may be made therein without departing from the spirit and scope of the present invention as defined by the following claims.
## Claims

1. A microarray substrate wherein a (poly)ethyleneglycol compound functionalized with an amino group is coated on a solid support having a polyanhydride surface, wherein (poly)ethylene glycols, amino groups and carboxyl groups are exposed on the surface of the microarray substrate.

2. The microarray substrate of claim 1, wherein the (poly)ethyleneglycol compound is a star-like or linear molecule.

3. The microarray substrate of claim 2, wherein the (poly)ethyleneglycol compound has a molecular weight of 60 to 10,000,000.

4. The microarray substrate of claim 1, wherein the polyanhydride is poly(ethylene-alt-maleic anhydride).

5. The microarray substrate of claim 4, wherein the poly(ethylene-alt-maleic anhydride) has a molecular weight of 128 to 10,000,000.

6. The microarray substrate of claim 1, wherein the solid support is made of a material selected from the group consisting of silicone, glass, and a plastic material.

7. The microarray substrate of claim 6, wherein the surface of the solid support is a solid support is coated with an amino group, a thiol group, or a hydroxy group-containing compound.

8. The microarray substrate of claim 7, wherein the solid support is a solid support coated with gamma-aminopropyltriethoxysilane, gamma-aminopropyldiethoxysilane, or aminohexyl.

9. The microarray substrate of claim 8, wherein the solid support is a solid support coated with gamma-aminopropyltriethoxysilane.

10. A method of manufacturing a microarray substrate according to claim 1, the method comprising:
(a) preparing a solid support coated with an amino group-containing compound;
(b) immobilizing a polyanhydride on a surface of the solid support;
(c) incorporating an amino group into an end of (poly)ethyleneglycol; and
(d) coating the polyanhydride-immobilized solid support with amino-functionalized (poly)ethyleneglycol, whereby the anhydride group of the polyanhydride reacts with an amino group of the functionalized (poly)ethylene glycol.

11. The method of claim 10, wherein in (c), the functionalized (poly)ethyleneglycol is a star-like or linear molecule.

12. The method of claim 10, wherein in (c), the functionalized (poly)ethyleneglycol has a molecular weight of 60 to 10,000,000.

13. The method of claim 10, wherein in (b), the polyanhydride is poly(ethylene-alt-maleic anhydride).

14. The method of claim 13, wherein the poly(ethylene-alt-maleic anhydride) has a molecular weight of 128 to 10,000,000.

15. The method of claim 10, wherein the solid support is made of a material selected from the group consisting of silicone, glass, and a plastic material.

16. The method of claim 15, wherein the amino group-containing compound is gamma-aminopropyltriethoxysilane, gamma-aminopropyldiethoxysilane, or aminohexyl.

17. A microarray wherein the microarray substrate of claim 1 is immobilized with a probe biomolecule.

18. The microarray of claim 17, wherein the probe biomolecule is a nucleic acid.

19. A method of analyzing a target biomolecule using the microarray of claim 17 or 18.

## Patentansprüche

1. Mikroarray-Substrat, wobei eine Polyethylenglykol-Verbindung, die mit einer Aminogruppe funktionalisiert ist, auf einem festen Träger, der eine Polyanhydridoberfläche aufweist, aufgetragen ist, wobei Polyethylenglykole, Aminogruppen und Carboxylgruppen an der Oberfläche von dem Mikroarray-Substrat exponiert sind.

2. Mikroarray-Substrat gemäß Anspruch 1, wobei die Polyethylenglykol-Verbindung ein sternförmiges oder lineares Molekül ist.

3. Mikroarray-Substrat gemäß Anspruch 2, wobei die Polyethylenglykol-Verbindung ein Molekulargewicht von 60 bis 10.000.000 aufweist.

4. Mikroarray-Substrat gemäß Anspruch 1, wobei das Polyanhydrid ein Poly(ethylen-alt-Maleinsäureanhydrid) ist.

5. Mikroarray-Substrat gemäß Anspruch 4, wobei das Poly(ethylen-alt-Maleinsäureanhydrid) ein Molekulargewicht von 128 bis 10.000.000 aufweist.

6. Mikroarray-Substrat gemäß Anspruch 1, wobei der feste Träger aus einem Material hergestellt ist, das ausgewählt ist aus der Gruppe bestehend aus Silikon, Glas und einem Kunststoff.

7. Mikroarray-Substrat gemäß Anspruch 6, wobei die Oberfläche von dem festen Träger mit einer eine Aminogruppe, eine Thiolgruppe oder eine Hydroxygruppe enthaltenden Verbindung beschichtet ist.

8. Mikroarray-Substrat gemäß Anspruch 7, wobei der feste Träger mit γ-Aminopropyltriethoxysilan, γ-Aminopropyldiethoxysilan oder Aminohexyl beschichtet ist.

9. Mikroarray-Substrat gemäß Anspruch 8, wobei der feste Träger mit γ-Aminopropyltriethoxysilan beschichtet ist.

10. Verfahren zum Herstellen eines Mikroarray-Substrates gemäß Anspruch 1, wobei das Verfahren umfasst:
(a) Bereitstellen eines festen Trägers, der mit einer eine Aminogruppe enthaltenden Verbindung beschichtet ist;
(b) Immobilisieren eines Polyanhydrides auf der Oberfläche von dem festen Träger;
(c) Einbauen einer Aminogruppe an einem Ende von einem Polyethylenglykol; und
(d) Beschichten des festen Trägers, auf dem das Polyanhydrid immobilisiert ist, mit dem Amino-funktionalisierten Polyethylenglykol, wobei die Anhydridgruppe von dem Polyanhydrid mit einer Aminogruppe von dem funktionalisierten Polyethylenglykol reagiert.

11. Verfahren gemäß Anspruch 10, wobei das funktionalisierte Polyethylenglykol aus Schritt (c) ein sternförmiges oder lineares Molekül ist.

12. Verfahren gemäß Anspruch 10, wobei das funktionalisierte Polyethylenglykol aus Schritt (c) ein Molekulargewicht von 60 bis 10.000.000 aufweist.

13. Verfahren gemäß Anspruch 10, wobei das Polyanhydrid aus Schritt (b) ein Poly(ethylen-alt-Maleinsäureanhydrid) ist.

14. Verfahren gemäß Anspruch 13, wobei das Poly(ethylen-alt-Maleinsäureanhydrid) ein Molekulargewicht von 128 bis 10.000.000 aufweist.

15. Verfahren gemäß Anspruch 10, wobei der feste Träger aus einem Material hergestellt ist, das ausgewählt ist aus der Gruppe bestehend aus Silikon, Glas und einem Kunststoff.

16. Verfahren gemäß Anspruch 15, wobei die eine Aminogruppe enthaltende Verbindung γ-Aminopropyltriethoxysilan, γ-Aminopropyldiethoxysilan oder Aminohexyl ist.

17. Mikroarray, wobei auf einem Mikroarray-Substrat gemäß Anspruch 1 ein Sonden-Biomolekül immobilisiert ist.

18. Mikroarray gemäß Anspruch 17, wobei das Sonden-Biomolekül eine Nukleinsäure ist.

19. Verfahren zum Analysieren eines Ziel-Biomoleküls unter Verwenden des Mikroarrays gemäß Anspruch 17 oder 18.

## Revendications

1. Substrat de microréseau où un composé de (poly)éthylèneglycol fonctionnalisé avec un groupe amino est appliqué sous forme de revêtement sur un support solide ayant une surface portant un polyanhydride, où des (poly)éthylèneglycols, des groupes amino et des groupes carboxyle sont exposés à la surface du substrat de microréseau.

2. Substrat de microréseau selon la revendication 1, dans lequel le composé de (poly)éthylèneglycol est une molécule linéaire ou en étoile.

3. Substrat de microréseau selon la revendication 2, dans lequel le composé de (poly)éthylèneglycol a un poids moléculaire de 60 à 10 000 000.

4. Substrat de microréseau selon la revendication 1, dans lequel le polyanhydride est un poly(éthylène-alt-anhydride maléique).

5. Substrat de microréseau selon la revendication 4, dans lequel le poly(éthylène-alt-anhydride maléique) a un poids moléculaire de 128 à 10 000 000.

6. Substrat de microréseau selon la revendication 1, où le support solide est fait d'un matériau choisi dans le groupe constitué d'un silicone, du verre et d'un matériau plastique.

7. Substrat de microréseau selon la revendication 6, où la surface du support solide est un support solide qui est revêtu d'un composé contenant un ou plusieurs groupes amino, thiol, ou hydroxy.

8. Substrat de microréseau selon la revendication 7, où le support solide est un support solide revêtu de gamma-aminopropyltriéthoxysilane, de gamma-aminopropyldiéthoxysilane, ou d'aminohexyle.

9. Substrat de microréseau selon la revendication 9, où le support solide est un support solide revêtu de gamma-aminopropyltriéthoxysilane.

10. Procédé de fabrication d'un substrat de microréseau selon la revendication 1, ledit procédé consistant à :
(a) préparer un support solide revêtu d'un composé contenant un ou plusieurs groupes amino ;
(b) immobiliser un polyanhydride sur une surface du support solide ;
(c) incorporer un groupe amino dans une extrémité d'un (poly)éthylèneglycol ; et
(d) revêtir le support solide sur lequel a été immobilisé le polyanhydride, avec le (poly)éthylèneglycol fonctionnalisé par un groupe amino, moyennant quoi le groupe anhydride du polyanhydride réagit avec un groupe amino du (poly)éthylèneglycol fonctionnalisé.

11. Procédé selon la revendication 10, dans lequel en (c), le (poly)éthylèneglycol fonctionnalisé est une molécule linéaire ou en étoile.

12. Procédé selon la revendication 10, dans lequel en (c), le (poly)éthylèneglycol fonctionnalisé a un poids moléculaire de 60 à 10 000 000.

13. Procédé selon la revendication 10, dans lequel en (b), le polyanhydride est un poly(éthylène-alt-anhydride maléique).

14. Procédé selon la revendication 13, dans lequel le poly(éthylène-alt-anhydride maléique) a un poids moléculaire de 128 à 10 000 000.

15. Procédé selon la revendication 10, dans lequel le support solide est fait d'un matériau choisi dans le groupe constitué d'un silicone, du verre et d'un matériau plastique.

16. Procédé selon la revendication 15, dans lequel le composé contenant un ou plusieurs groupes amino est un gamma-aminopropyltriéthoxysilane, un gamma-aminopropyldiéthoxysilane, ou un aminohexyle.

17. Microréseau où le substrat de microréseau selon la revendication 1 est immobilisé avec une biomolécule servant de sonde.

18. Microréseau selon la revendication 17, où la biomolécule servant de sonde est un acide nucléique.

19. Procédé d'analyse d'une biomolécule cible utilisant le microréseau selon la revendication 17 ou 18.
